# EUROPÄISCHE PATENTANMELDUNG

(11) **EP 2 478 836 A1**
(43) Veröffentlichungstag der Anmeldung: **25.07.2012**
(21) Anmeldenummer: 11151877.5
(22) Anmeldetag: 24.01.2011
(51) Int. Cl.: A61B 5/0255, A61B 5/024

(54) **Verfahren und Vorrichtung zur Visualisierung einer Mehrzahl von Puls-Signalen**

(71) Anmelder: Hendricks, Markus, 22765 Hamburg (DE)
(72) Erfinder: Hendricks, Markus, 22765 Hamburg (DE)

(57) **Zusammenfassung**

Die vorliegende Erfindung betrifft ein Verfahren zur Visualisierung einer Mehrzahl von Puls-Signalen mit den Schritten:
- Empfangen mindestens eines ersten Puls-Signals der Mehrzahl von Puls-Signalen,
- Visualisieren einer quantifizierbaren Eigenschaft des mindestens einen ersten Puls-Signals auf einem Anzeigemittel (21),

dadurch gekennzeichnet, dass das Verfahren ferner die Schritte aufweist:
- Speichern der quantifizierbaren Eigenschaft des mindestens einen ersten Puls-Signals,
- Empfangen mindestens eines zweiten Puls-Signals der Mehrzahl von Puls-Signalen, und
- Zuordnen des mindestens einen ersten Puls-Signals und des mindestens einen zweiten Puls-Signals jeweils zu einem bestimmten Körpermesspunkt und/oder Messdruck, und dadurch gekennzeichnet, dass

die quantifizierbare Eigenschaft des mindestens einen ersten Puls-Signals auf dem Anzeigemittel (21) visualisiert wird, indem die gespeicherte quantifizierbare Eigenschaft des mindestens einen ersten Puls-Signals und die quantifizierbare Eigenschaft des mindestens einen zweiten Puls-Signals jeweils an einer für die Zuordnung zu einem Körpermesspunkt und/oder Messdruck charakteristischen Stelle auf dem Anzeigemittel (21) visualisiert werden.

## Beschreibung

Die vorliegende Erfindung betrifft ein Verfahren und eine Vorrichtung zur Visualisierung einer Mehrzahl von Puls-Signalen gemäß dem Oberbegriff des Anspruchs 1 bzw. 8.

Sowohl in der traditionellen chinesischen Medizin (TCM) als auch in der graeco-arabischen Medizin (Unani), der ayurvedischen indischen Medizin sowie in ganzheitlichen Ansätzen der westlichen Schulmedizin ist die Pulsdiagnose ein integraler Bestandteil. Die TCM kennt dabei im Unterschied zur westlichen Schulmedizin beispielsweise verschiedene Qualitäten des menschlichen Pulses an verschiedenen Körpermesspunkten. Ayurveda-Mediziner überprüfen bei der Pulsdiagnose Lebensenergien eines Patienten und ermitteln vorliegende "Störungen". Meist unterscheidet man bei der Pulsdiagnose die Körperpulse vom Handgelenkspuls (Radialispuls).

Heutzutage beschränkt sich das manuelle Tasten der Pulse allerdings fast ausschließlich auf die Handgelenke. Die Messung des Handgelenkpulses (*cun kou*) findet an den drei Körpermesspunkten *cun*, *guan* und *chi*, die nebeneinander über der Arteria radialis am Handgelenk liegen, an beiden Händen mit verschiedenen Messdrücken statt. Man orientiert sich dabei am Griffelfortsatz der Speiche, dem Processus styloideus radii. Etwas nach ulnar befindet sich zwischen dem Griffelfortsatz und der Sehne des speichenseitigen Handbeugers die Radialisarterie. An dieser Stelle wird bei der manuellen Pulsdiagnose der Mittelfinger der Behandlungsperson platziert, der sich dann in der *guan*-Position befindet. Wenn nun Zeige-und Ringfinger am Mittelfinger anliegend jeweils proximal und distal davon angelegt werden, liegt der Ringfinger proximal auf der *chi*-Position und der Zeigefinger distal auf der *cun-*Position. Der Messdruck wird bei der manuellen Pulsdiagnose meist qualitativ in die Messdrücke *oberflächig* und *tief* eingeteilt.

Aus der Pulsfrequenz (schnell oder langsam), dem Rhythmus (regelmäßig oder unregelmäßig), der Pulskraft (voll oder leer), der Pulsform (dünn oder breit, kurz oder lang), der Pulskonsistenz (weich oder hart), der Pulsqualität (gleitend, gespannt, etc.) oder anderen Eigenschaften kann eine behandelnde Person mit sehr viel Erfahrung in fernöstlichen Behandlungsmethoden eine qualitative Einteilung in eines von etwa 29 verschiedenen Pulsbildern vornehmen und daraus ein bestimmtes Krankheitsbild oder eine Diagnose ableiten.

Vor diesem Hintergrund beschreibt die US 4,066,066 A1 beispielsweise ein elektrisches Pulsgerät zur Unterstützung einer medizinisch behandelnden Person bei einer Diagnose nach fernöstlicher Medizin durch Ausdrucke von Wellenformen mehrerer verschiedener menschlicher Blutgefäßpulse. Die produzierten Wellenformen können dann durch die medizinisch behandelnde Person interpretiert werden und mit den Pulsbeschreibungen in den alten Büchern der fernöstlichen Medizin verglichen werden. Dadurch wird die medizinisch behandelnde Person unterstützt, ein Krankheitsbild generell zu lokalisieren und zu einer Diagnose zu gelangen.

Nachteilig an der beschriebenen Vorrichtung ist allerdings, dass nach mehreren Messungen an verschiedenen Körpermesspunkten mit jeweils verschiedenen Messdrücken eine Vielzahl von niedergeschriebenen oder ausgedruckten Wellenformen vorliegt und das Auswerten der Wellenformen mühsam und langwierig ist. Ein Vergleich der Wellenformen mit Pulsbeschreibungen in den alten Büchern der fernöstlichen Medizin ist in der Praxis sehr schwierig, da sich nur mit sehr viel Erfahrung bestimmte Erkenntnisse aus den Wellenformen gewinnen lassen.

Es ist daher die Aufgabe der vorliegenden Erfindung, ein Verfahren und eine Vorrichtung zur Visualisierung einer Mehrzahl von Puls-Signalen bereitzustellen, das die Nachteile des Standes der Technik überwindet und die Auswertung der gemessenen Puls-Signale wesentlich vereinfacht.

Gelöst wird diese Aufgabe durch ein Verfahren und eine Vorrichtung zur Visualisierung einer Mehrzahl von Puls-Signalen gemäß den Merkmalen des Anspruchs 1 bzw. 8. Vorteilhafte Ausführungsformen der Erfindung sind den jeweiligen Unteransprüchen bzw. der Beschreibung und den Figuren zu entnehmen.

Gemäß einem ersten Aspekt der Erfindung wird ein Verfahren zur Visualisierung einer Mehrzahl von Puls-Signalen mit den Schritten:
- Empfangen mindestens eines ersten Puls-Signals der Mehrzahl von Puls-Signalen,
- Visualisieren einer quantifizierbaren Eigenschaft des mindestens einen ersten Puls-Signals auf einem Anzeigemittel,
bereitgestellt, das dadurch gekennzeichnet ist, dass das Verfahren ferner die Schritte aufweist:
- Speichern der quantifizierbaren Eigenschaft des mindestens einen ersten Puls-Signals,
- Empfangen mindestens eines zweiten Puls-Signals der Mehrzahl von Puls-Signalen, und
- Zuordnen des mindestens einen ersten Puls-Signals und des mindestens einen zweiten Puls-Signals jeweils zu

einem bestimmten Körpermesspunkt und/oder Messdruck, und das ferner dadurch gekennzeichnet ist, dass die quantifizierbare Eigenschaft des mindestens einen ersten Puls-Signals auf dem Anzeigemittel visualisiert wird, indem die gespeicherte quantifizierbare Eigenschaft des mindestens einen ersten Puls-Signals und die quantifizierbare Eigenschaft des mindestens einen zweiten Puls-Signals jeweils an einer für die Zuordnung zu einem Körpermesspunkt und/oder Messdruck charakteristischen Stelle auf dem Anzeigemittel visualisiert wird.

Es sei an dieser Stelle angemerkt, dass es sich bei dem vorliegenden Verfahren nicht um ein Diagnostizierverfahren handelt, sondern um ein technisches Verfahren zur Visualisierung einer Mehrzahl von Puls-Signalen, sodass die Auswertung der Puls-Signale vereinfacht, aber nicht durch das Verfahren selbst ausgeführt wird. Die zeitliche Reihenfolge der Verfahrensschritte ist dabei nicht durch die Auflistung der Schritte oder die Nummerierung der Puls-Signale indiziert oder beschränkt. Die Verfahrensschritte können gleichzeitig oder in beliebiger Reihenfolge ausgeführt werden.

Unter dem Begriff "Körpermesspunkt" ist hierin der Ort auf der Haut eines Lebewesens zu verstehen, wo ein Puls gemessen wird oder wurde. Unter dem Begriff "Messdruck" ist hierin der Anpressdruck auf die Haut eines Lebewesens zu verstehen, der zur Messung des Pulses auf die Haut ausgeübt wird.

Das erfindungsgemäße Verfahren hat den Vorteil, dass eine Mehrzahl von Puls-Signalen übersichtlich dargestellt werden kann und die behandelnde Person leicht alle Puls-Signale auf einem Blick miteinander vergleichen kann. Im Falle einer Pulsdiagnose nach TCM können beispielsweise die maximalen Signalamplituden von 12 Puls-Signalen nebeneinander angezeigt werden, die an den drei Körpermesspunkten *chi*, *guan* und *cun* am Handgelenk jeder Hand mit den jeweiligen Messdrücken *oberflächig* und *tief* gemessen wurden bzw. werden.

Vorzugsweise ist die quantifizierbare Eigenschaft die maximale Signalamplitude, aber es kann auch eine beliebige andere quantifizierbare Eigenschaft visualisiert werden, wie etwa die Pulsfrequenz, ein Integral unter der Pulskurve zwischen zwei Messzeiten, die mittlere Signalamplitude, gewisse Flankensteigungen, usw. Auch qualitative Eigenschaften wie etwa die Pulsqualität (gleitend, gespannt, etc.) lassen sich mit geeigneten Analyseverfahren, die auf die Puls-Signale angewendet werden, in Form einer Zuordnung zu einem einer definierten Anzahl von Pulsbildern als quantifizierbare Eigenschaft visualisiert werden.

Vorzugsweise werden die Signalamplituden der Mehrzahl von Puls-Signalen jeweils in Bezug auf eine Skala visualisiert, wobei das Maximum der Skala durch das Puls-Signal mit der höchsten maximalen Signalamplitude bestimmt wird und das Minimum der Skala durch das Puls-Signal mit der niedrigsten maximalen Signalamplitude bestimmt wird.

Dies hat den Vorteil, dass die Puls-Signale relativ zueinander in einfacher Weise verglichen werden können. Die behandelnde Person sieht beispielsweise auf einen Blick ein Ungleichgewicht zwischen dem Puls an der linken und dem entsprechenden Puls an der rechten Hand. Die Darstellung sowohl der gespeicherten Daten (z.B. erste Puls-Signale) als auch der momentan empfangenen Daten (z.B. zweite Puls-Signale) kann sich dadurch während des Empfangens weiterer Puls-Signale ständig ändern. Sobald sich alle Werte stabilisiert haben, kann die gesamte Darstellung für eine Diagnose herangezogen werden.

Vorzugsweise wird das mindestens eine zweite Puls-Signal in Echtzeit visualisiert, d.h. das Visualisieren der quantifizierbaren Eigenschaft des mindestens einen zweiten Puls-Signals wird vorzugsweise vor dem Ende des Empfangens des mindestens einen zweiten Puls-Signals begonnen. Dadurch erhält die Behandlungsperson sofort einen optischen Eindruck von dem momentan empfangenen zweiten Puls-Signal und kann einen Vergleich mit dem gespeicherten und ebenfalls dargestellten ersten Puls-Signal vornehmen.

Es ist besonders bevorzugt, die Mehrzahl von Puls-Signalen in eine oder mehrere Gruppen einzuteilen und die Summe und/oder den Durchschnittswert der quantifizierbaren Eigenschaft der Puls-Signale jeder Gruppe jeweils an einer für die Gruppe charakteristischen Stelle auf dem Anzeigemittel zu visualisieren. Eine Gruppe kann dabei auch ein einzelnes oder auch sämtliche der Mehrzahl von Puls-Signalen umfassen.

Diese Gruppeneinteilung erleichtert beispielweise die sehr häufig bei der Pulsdiagnose nach TCM angewendete Zuordnung der Pulse zur fernöstlichen Fünf-Elemente-Lehre, die zur Findung eines Krankheitsbildes oder einer Diagnose herangezogen werden kann. Dabei kann zum Beispiel die folgende Zuordnung eine Rolle spielen:

| **Linke Hand** | **Messdruck** | **Element** | **Farbe** | **Organ** |
|---|---|---|---|---|
| cun | tief | Feuer | rot | Herz |
| | oberflächlich | | | Dünndarm |
| guan | tief | Holz | grün | Leber |
| | oberflächlich | | | Gallenblase |
| chi | tief | Wasser | blau | Niere |
| | oberflächlich | | | Harnblase |

| **Rechte Hand** | **Messdruck** | **Element** | **Farbe** | **Organ** |
|---|---|---|---|---|
| cun | tief | Metall | silber grau/ | Lunge |
| | oberflächlich | | | Dickdarm |
| guan | tief | Erde | braun | Milz |
| | oberflächlich | | | Magen |
| chi | tief | Feuer | rot | Pericard |
| | oberflächlich | | | 3-Erwärmer |

Zum Beispiel können die Summe und/oder der Durchschnittswert der maximalen Signalamplituden der jeweils tief und oberflächlich gemessenen Pulse an der zugehörigen Ecke eines Fünf-Elemente-Fünfecks dargestellt werden. Die an der *cun-*Position gemessenen Pulse der linken Hand und die an der chi-Position gemessenen Pulse der rechten Hand können beispielsweise ebenfalls zu einer Summe und/oder einem Durchschnittswert zusammengefasst werden, da sie das gleiche Element (Feuer) betreffen. Der relative Anteil des jeweils tief gemessenen Pulses (sogenanntes Yin-Gewicht) und des jeweils oberflächlich gemessenen Pulses (sogenanntes Yang-Gewicht) ist für jede Summe und/oder jeden Durchschnittswert darstellbar und kann beispielsweise an der entsprechenden Ecke des Fünf-Elemente-Fünfecks visualisiert werden. Dadurch wird ein Ungleichgewicht der Pulse besonders deutlich dargestellt, sodass die Diagnose schneller und einfacher von der behandelnden Person vorgenommen werden kann.

Vorzugsweise kann dabei jeder Gruppe eine für die Gruppe charakteristische Farbe und/oder Bezeichnung zugeordnet werden und die Summe und/oder der Durchschnittswert der quantifizierbaren Eigenschaft der Puls-Signale jeder Gruppe jeweils in der für die Gruppe charakteristischen Farbe und/oder mit der für die Gruppe charakteristischen Bezeichnung auf dem Anzeigemittel visualisiert werden.

Zusätzlich kann beispielsweise mit der Gewichtung der Summe und/oder des Durchschnittswerts der jeweiligen Gruppe eine Gesamtmischfarbe in der Mitte des Fünf-Elemente-Fünfecks erzeugt werden, was der behandelnden Person auf einen Blick einen optischen Gesamteindruck vom Zustand der behandelten Person verschafft, denn Ungleichgewichte der Pulse in eine Richtung des Fünf-Elemente-Fünfecks führen unverzüglich zu einer spezifischen Farbänderung der Gesamtmischfarbe.

Es ist darüber hinaus vorteilhaft, wenn der Summe und/oder dem Durchschnittswert der quantifizierbaren Eigenschaft der Puls-Signale einer Gruppe ein Audiosignal zugeordnet wird und das Audiosignal über einen Audio-Ausgang oder einen Lautsprecher ausgegeben wird. Damit kann beispielsweise ein charakteristischer Ton der behandelnden Person ein akustisches Bild vom Gesundheitszustand vermitteln. Für blinde Behandlungspersonen und/oder Patienten kann dies besonders hilfreich sein. Aber auch für Hörende kann ein Ton häufig leichter beispielsweise als harmonisch empfunden werden als ein gefühlter Puls oder eine Mischfarbe. Darüber hinaus kann ein Ausgabeton nicht nur zu Diagnosezwecken verwendet werden, sondern auch zu Heilungszwecken. Die Umrechnung von Farbe in Klang kann nach dem physikalischen Prinzip der Oktavierung nach Hans Cousto erfolgen und somit oktavanalog sein. Dabei wird die Schwingungsfrequenz der Farbe so oft durch zwei geteilt bis der hörbare Frequenzbereich erreicht wird. Innerhalb des hörbaren Bereichs werden die Oktaven zu einem Tonsignal gemischt. Sofern ein Ungleichgewicht der Pulse festgestellt wird, kann über eine Klangtherapie ein nach dem oben geschriebenen Prinzip erzeugter Ton dazu führen, dass sich wieder ein Gleichgewicht herstellt. Dies kann auch während der Pulsmessung erfolgen und man kann den Verlauf der Besserung sogar verfolgen. Die Klangtherapie durch den Ausgabeton kann ständig der verbesserten Gleichgewichtslage angepasst werden bis ein ausreichendes Gleichgewicht hergestellt ist. Nicht nur ein Ausgabeton kann hier eingesetzt werden, sondern auch andere Schwingungen wie etwa eine Lichttherapie mit entsprechender Farbe oder andere elektromagnetische Schwingungen mit entsprechender Wellenlänge.

Gemäß einem zweiten Aspekt der Erfindung wird eine Vorrichtung zur Visualisierung einer Mehrzahl von Puls-Signalen mit
- einem Puls-Signalverarbeitungsmittel, das dazu eingerichtet ist, mindestens ein erstes Puls-Signal der Mehrzahl von Puls-Signalen zu empfangen, und
- einem Anzeigemittel, das dazu eingerichtet ist, eine quantifizierbare Eigenschaft des mindestens einen ersten Puls-Signals auf einem Anzeigemittel zu visualisieren,
bereitgestellt, das dadurch gekennzeichnet ist, dass das Puls-Signalverarbeitungsmittel ferner dazu eingerichtet ist, die quantifizierbare Eigenschaft des mindestens einen ersten Puls-Signals zu speichern, mindestens ein zweites Puls-Signal der Mehrzahl von Puls-Signalen zu empfangen, und das mindestens eine erste Puls-Signal und das mindestens eine zweite Puls-Signal jeweils einem bestimmten Körpermesspunkt und/oder Messdruck zuzuordnen, und
dass das Anzeigemittel ferner dazu eingerichtet ist, die quantifizierbare Eigenschaft des mindestens einen ersten Puls-Signals auf dem Anzeigemittel zu visualisieren, indem die gespeicherte quantifizierbare Eigenschaft des mindestens einen ersten Puls-Signals und die quantifizierbare Eigenschaft des mindestens einen zweiten Puls-Signals jeweils an einer für die Zuordnung zu einem Körpermesspunkt und/oder Messdruck charakteristischen Stelle auf dem Anzeigemittel visualisiert wird.

Das Anzeigemittel weist dabei vorzugsweise ein Farbdisplay auf. Es sei an dieser Stelle darauf hingewiesen, dass das Anzeigemittel auch weitere Hardware- bzw. Softwarekomponenten aufweisen kann wie etwa einen Prozessor, RAM, Grafikbausteine, Speicherbausteine, Treiber, usw. Diese Hardware- bzw. Softwarekomponenten können ausschließlich zu dem Anzeigemittel gehören oder auch Teil des Puls-Signalverarbeitungsmittels oder anderer Komponenten der Vorrichtung sein.

Vorzugsweise ist die erfindungsgemäße Vorrichtung dazu eingerichtet, dass das mindestens eine zweite Puls-Signal in Echtzeit visualisiert wird, d.h. dass das Visualisieren der quantifizierbaren Eigenschaft des mindestens einen zweiten Puls-Signals vorzugsweise vor dem Ende des Empfangens des mindestens einen zweiten Puls-Signals begonnen wird. Dadurch kann die Behandlungsperson sofort einen optischen Eindruck von dem momentan empfangenen zweiten Puls-Signal erhalten und einen Vergleich mit dem gespeicherten und ebenfalls dargestellten ersten Puls-Signal vornehmen.

Die erfindungsgemäße Vorrichtung weist vorzugsweise ferner ein mit dem Puls-Signalverarbeitungsmittel kommunikativ verbundenes Puls-Signalerfassungsmittel auf, das dazu eingerichtet ist, gleichzeitig den Puls an drei nebeneinander liegenden Körpermesspunkten zu messen.

Das Puls-Signalerfassungsmittel weist dabei vorzugsweise drei in einer Reihe angeordnete Drucksensoren auf, wobei das Signalerfassungsmittel dazu eingerichtet ist, den Puls bei mindestens zwei verschiedenen reproduzierbaren Messdrücken zu messen. Das Puls-Signalerfassungsmittel kann beispielsweise eine Handgelenksmanschette sein, die innenseitig drei in einer Reihe angeordnete Piezosensoren aufweist, die mittels der Handgelenksmanschette mit einem reproduzierbaren Messdruck auf die Körpermesspunkte *cun*, *guan* und chi am Handgelenk gedrückt werden können. Die Handgelenksmanschette ist dazu vorzugsweise mit einem reproduzierbaren Luftdruck aufblasbar.

Es ist besonders bevorzugt, wenn das Puls-Signalverarbeitungsmittel einen Audio-Eingang aufweist, der dazu eingerichtet ist, die Puls-Signale zu empfangen. Dies ermöglicht es, dass das Puls-Signalverarbeitungsmittel nicht nur ein spezielles elektronisches Gerät, sondern auch ein für andere Zwecke verwendbares elektronisches Gerät sein kann wie etwa ein PC, Notebook, Netbook, Handy, Smartphone, Palm, Tablett-Computer oder Ähnliches. Solch ein Gerät kann auch das Anzeigemittel umfassen, beispielsweise mit einem Farbdisplay, oder damit verbunden sein. Das erfindungsgemäße Verfahren kann dann in einfacher Weise in Form einer installierbaren Software-Applikation zur Steuerung der Visualisierung der über den Audio-Eingang empfangenen Puls-Signale auf einem Display ausgeführt werden. Ein elektronisches Gerät mit einer solchen installierten Software-Applikation stellt eine bevorzugte Ausführungsform der erfindungsgemäßen Vorrichtung dar. Vorzugsweise weist der Audio-Eingang mindestens drei Kanäle auf, damit mindestens die drei Audio-Signale von der cun-, guan- und chi-Position gleichzeitig empfangen werden können.

Vorzugsweise weist das Puls-Signalverarbeitungsmittel einen Audio-Ausgang oder Lautsprecher auf, der dazu eingerichtet ist, ein für die quantifizierbare Eigenschaft der empfangenen und/oder gespeicherten Puls-Signale zugeordnetes Audiosignal auszugeben.

Die vorliegende Erfindung kann in beliebiger Weise durch Hardware- und/oder Software-Komponenten realisiert werden. Die Erfindung umfasst daher auch jegliches maschinen-lesbare Medium mit gespeicherten Anweisungen zur Ausführung der erfindungsgemäßen Verfahrensschritte oder zum Betrieb einer erfindungsgemäßen Vorrichtung.

Im Folgenden wird die Erfindung anhand der beiliegenden Figuren näher erläutert, die eine bevorzugte Ausführungsform der Erfindung zeigen. In den Figuren ist im Einzelnen zu erkennen:
- Fig. 1:: Eine schematische Darstellung der manuellen Pulsdiagnose am Handgelenk nach TCM;
- Fig. 2:: Eine schematische Darstellung einer Ausführungsform der erfindungsgemäßen Vorrichtung;
- Fig. 3:: Eine Querschnittsansicht durch eine Handgelenksmanschette eines Puls-Signalerfassungsmittels einer Ausführungsform der erfindungsgemäßen Vorrichtung;
- Fig. 4a,b:: Längsschnittsansichten durch einen Piezo-Sensor eines Puls-Signalerfassungsmittels einer Ausführungsform der erfindungsgemäßen Vorrichtung im Ausgangszustand bzw. eingedrücktem Zustand;
- Fig. 4c,d:: Eine Querschnittsansicht durch einen Piezo-Sensor eines Puls-Signalerfassungsmittels einer Ausführungsform der erfindungsgemäßen Vorrichtung im Ausgangszustand bzw. eingedrücktem Zustand; und
- Fig. 5:: Eine detaillierte Darstellung eines Farbdisplays eines Anzeigemittels einer Ausführungsform der erfindungsgemäßen Vorrichtung.

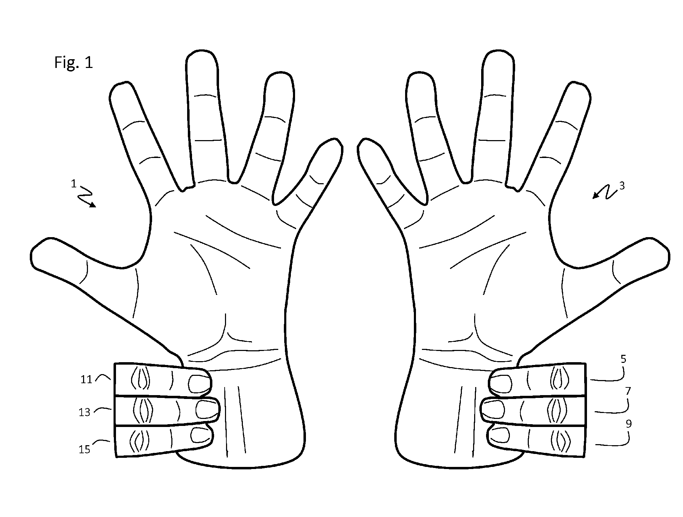

In Fig. 1 zeigt eine linke Hand 1 und eine rechte Hand 3 eines Patienten, der sich einer manuellen Pulsdiagnose nach traditioneller chinesischer Medizin (TCM) am Handgelenk unterzieht und einer sich ihm gegenüber befindlichen Behandlungsperson die nach oben geöffneten Handflächen entgegenstreckt. Die Behandlungsperson greift mit dem Zeigefinger 5, Mittelfinger 7 und Ringfinder 9 ihrer linken Hand an das Handgelenk der rechten Hand 3 des Patienten und mit dem Zeigefinger 11, Mittelfinger 13 und Ringfinder 15 ihrer rechten Hand an das Handgelenk der linken Hand 1 des Patienten. Dabei liegen die Mittelfinger 7, 13 jeweils auf der sogenannten guan-Position. Die guan-Position liegt auf der Radialisarterie zwischen dem Griffelfortsatz der Speiche, dem Processus styloideus radii, und der Sehne des speichenseitigen Handbeugers. Am Mittelfinger 7, 13 anliegend befinden sich die Zeigefinder 5, 11 distal von der guan-Position auf der cun-Position und die Ringfinger 9, 15 entsprechend proximal auf der chi-Position.

Mit viel Erfahrung in fernöstlichen Behandlungsmethoden kann die Behandlungsperson bei verschiedenen Messdrücken (z.B. oberflächig und tief) aus der gefühlten Pulsfrequenz (z.B. schnell oder langsam), dem Rhythmus (z.B. regelmäßig oder unregelmäßig), der Pulskraft (z.B. voll oder leer), der Pulsform (z.B. dünn oder breit, kurz oder lang), der Pulskonsistenz (z.B. weich oder hart), der Pulsqualität (z.B. gleitend, gespannt, etc.) eine qualitative Einteilung in eines von etwa 29 verschiedenen Pulsbildern vornehmen und daraus ein bestimmtes Krankheitsbild oder eine Diagnose ableiten.

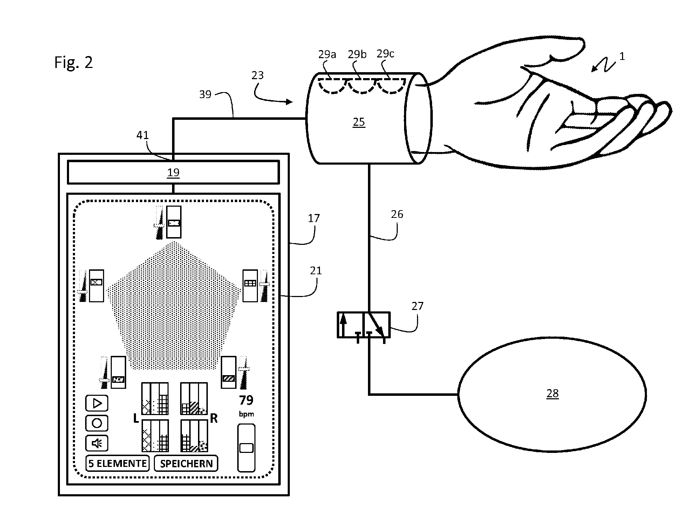

Fig. 2 zeigt eine schematische Darstellung einer Ausführungsform der erfindungsgemäßen Vorrichtung, mit der es einer Behandlungsperson erheblich vereinfacht wird, aus einer Mehrzahl von Pulssignalen ein bestimmtes Krankheitsbild oder eine Diagnose abzuleiten. Die Vorrichtung weist zunächst ein elektrisches Gerät 17 mit einem Puls-Signalverarbeitungsmittel 19 und einem Anzeigemittel 21 auf. Außerdem weist die Vorrichtung ein Puls-Signalerfassungsmittel 23 mit einer röhrenförmigen Handgelenksmanschette 25 auf, die über einen Schlauch 26 mit Wegeventil 27 durch eine Handpumpe 28 mit einem reproduzierbaren Luftdruck aufblasbar ist. Es versteht sich, dass es sich hierbei nicht um eine Handpumpe 28 handeln muss, sondern auch eine elektrische Pumpe oder jede andere Art von Pumpe dazu verwendet werden kann, einen reproduzierbaren Luftdruck bereitzustellen. Dabei kann beispielsweise eine automatische Druckregelung vorgesehen sein oder eine Druckanzeige als Orientierung zur Reproduktion eines Messdrucks dienen. Es kann aber auch einfach über die Anzahl der Pumpzyklen, z.B. bei einer Handpumpe 28 ohne Druckanzeige, ein Messdruck in ausreichendem Maße reproduziert werden.

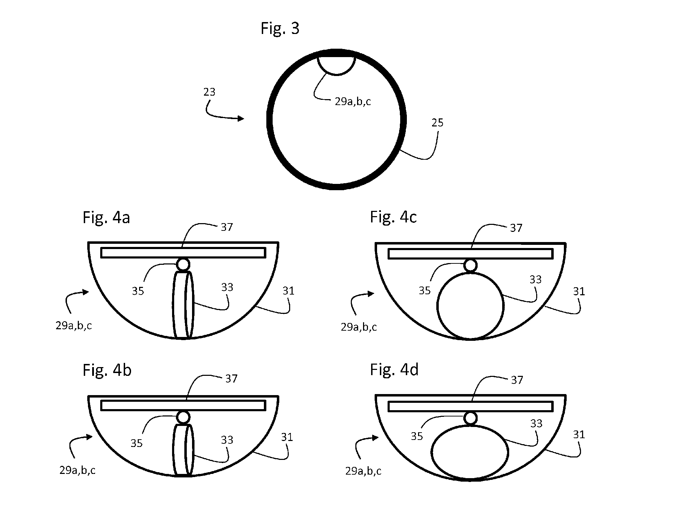

Innenseitig an der aufblasbaren Handgelenksmanschette 25 weist das Puls-Signalerfassungsmittel 23 drei in einer Reihe angeordnete Piezo-Sensoren 29a, 29b, 29c auf, wobei der distale Piezo-Sensor 29a in der cun-Position, der mittlere Piezo-Sensor 29b in der guan-Position und der proximale Piezo-Sensor 29c in der chi-Position durch den Luftdruck in der angelegten Handgelenksmanschette 25 mit einem bestimmten Messdruck auf die Radialisarterie gedrückt wird. Fig. 3 zeigt einen schematischen Querschnitt durch die Handgelenksmanschette 25 und einen der Piezo-Sensoren 29a, 29b, 29c.

Wie in den Figuren 4a-d genauer gezeigt, weisen die Piezo-Sensoren 29a, 29b, 29c selbst jeweils eine fingerkuppenförmige konvexe äußere Form auf, die sich flexibel eindrücken lässt. Dazu weist die fingerkuppenförmige konvexe äußere Form ein flexibles hautverträgliches Material 31 auf, wie etwa medizinisches Silikon. Innenseitig spannt eine Feder, beispielsweise in Form eines O-förmigen Federstahlbandes 33, das flexible hautverträgliche Material 31 zur fingerkuppenförmigen konvexen äußeren Form auf. Das Federstahlband 33 stützt sich dabei über einen Kegel oder eine Kugel, z.B. eine Metallkugel 35, gegen eine Piezoscheibe 37 mit Piezokristall ab. Wird bei angelegter Handgelenksmanschette 25 durch den Blutdruck in der Radialisarterie ein Druck auf die fingerkuppenförmige konvexe äußere Form ausgeübt, so gibt das Federstahlband 33 den Druck möglichst punktuell über die Metallkugel 35 auf den Piezokristall 37 weiter. Die durch den Druck am Piezokristall entstehende Spannung kann dann als Puls-Signal erfasst und verarbeitet werden.

Wie Fig. 2 zeigt, sind die Piezo-Sensoren 29a, 29b, 29c über eine Signalverbindung 39 kommunikativ mit dem Puls-Signalverarbeitungsmittel 19 des elektrischen Geräts 17 verbunden. Diese Signalverbindung 39 kann eine Kabelverbindung oder eine kabellose Verbindung sein. Das Puls-Signalverarbeitungsmittel 19 weist einen Audio-Eingang 41 auf, über den die Signalverbindung 39 die Puls-Signale in das Puls-Signalverarbeitungsmittel 19 einspeist. Sowohl die Signalverbindung 39 als auch der Audio-Eingang 41 sind mit drei Kanälen ausgestattet, damit die drei Audio-Signale von den drei Piezo-Sensoren 29a, 29b, 29c gleichzeitig empfangen werden können.

Das Puls-Signalverarbeitungsmittel 19 empfängt über den Audio-Eingang 41 mindestens ein erstes Puls-Signal einer Mehrzahl von Puls-Signalen, vorzugsweise die drei Puls-Signale der Piezo-Sensoren 29a, 29b, 29c. Eine quantifizierbare Eigenschaft der ersten Puls-Signals, wie etwa die maximale Signalamplitude innerhalb eines bestimmten Zeitraums, wird gespeichert. Der Zeitraum kann vorgegeben sein oder durch die Behandlungsperson bestimmt werden. Beispielsweise kann die Behandlungsperson über eine Eingabe-Schnittstelle den Befehl geben, die momentan jeweils empfangene maximale Signalamplitude zu speichern. Solch eine Eingabe-Schnittstelle kann beispielsweise durch einen Touch-Screen des Anzeigemittels 21 bereitgestellt werden und/oder durch andere Eingabemittel wie etwa eine Tastatur, eine Maus, einen Joystick oder Ähnliches. Nach dem Speichern der ersten Puls-Signale kann die Handpumpe 28 um eine bestimmte Anzahl von Pumpzyklen betätigt werden, um bei einem höheren Messdruck zweite Puls-Signale zu messen. Man kann auch durch Öffnen des Wegeventils 27 den Luftdruck in der Handgelenksmanschette 25 absenken, um bei einem niedrigeren Messdruck zweite Puls-Signale zu messen. Der Messdruck kann genau gemessen und dargestellt werden und/oder mittels definierter Schwellenwerte in oberflächig, d.h. niedrigen Messdrücken unterhalb eines Schwellenwerts, oder tief, d.h. hohen Messdrücken oberhalb eines Schwellenwerts, eingeteilt werden. Die Behandlungsperson kann auch über eine Eingabe-Schnittstelle dem Puls-Signalverarbeitungsmittel 19 mitteilen, welcher Messdruck den ersten bzw. zweiten Puls-Signalen zuzuordnen ist. Außerdem kann die Behandlungsperson über die Eingabe-Schnittstelle dem Puls-Signalverarbeitungsmittel 19 mitteilen, ob die ersten bzw. zweiten Puls-Signale an der linken bzw. rechten Hand gemessen wurden bzw. werden. Das Puls-Signalverarbeitungsmittel 19 führt eine entsprechende Zuordnung der Puls-Signale zu dem entsprechenden Körpermesspunkt und/oder Messdruck durch.

Die erfindungsgemäße Vorrichtung kann auch zwei oder mehr Puls-Signalverarbeitungsmittel aufweisen, beispielsweise mit einer zweiten Handgelenksmanschette. Damit kann die linke und die rechte Hand gleichzeitig gemessen werden. Die Zuordnung der jeweiligen Puls-Signale kann dann dadurch erfolgen, dass über entsprechend zugeordnete unterschiedliche Signal-Eingänge die Puls-Signale empfangen werden.

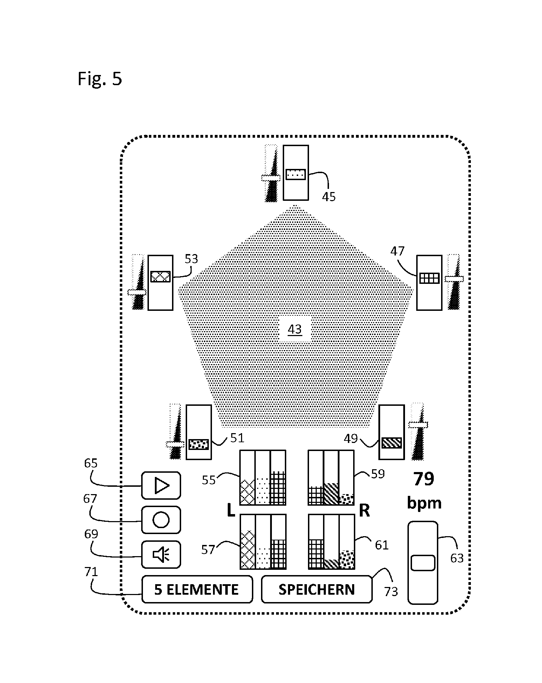

Das Anzeigemittel 21 ist mit dem Puls-Signalverarbeitungsmittel 19 in einer Einheit integriert und/oder mit dem Puls-Signalverarbeitungsmittel 19 kommunikativ verbunden. Die gespeicherte maximale Signalamplitude der ersten Puls-Signale und die maximale Signalamplitude der zweiten Puls-Signals werden durch das Anzeigemittel 21 auf einem Farbdisplay in Form eines Touch-Screens an einer für die Zuordnung zu dem jeweiligen Körpermesspunkt und/oder Messdruck charakteristischen Stelle auf dem Farbdisplay visualisiert. Dies ist in Fig. 5 genauer erkennbar, die das farbige Touch-Screen des Anzeigemittels 21 detaillierter darstellt.

Im oberen Bereich des Touch-Screens ist ein Fünf-Elemente-Fünfeck 43 dargestellt, wobei sich an der oberen Ecke 45 in grüner Farbe das Element "Holz" befindet. Rechts oben 47 ist in roter Farbe das Element "Feuer", rechts unten 49 in brauner Farbe das Element "Erde", links unten 51 in silber/grauer Farbe das Element "Metall" und links oben 53 in blauer Farbe das Element "Wasser". An jeder Ecke zeigt in der jeweiligen Farbe ein erster Balken auf einer Skala eine Gewichtung der Farbe in Bezug auf die Gesamtmessung an und ein zweiter Balken eine relative Gewichtung des jeweils tief (Yin) bzw. oberflächlich (Yang) gemessenen Pulses für die jeweilige Farbe. Das Fünf-Elemente-Fünfeck 43 ist mit einer Mischfarbe gefüllt, die einer Farbmischung aus den fünf Farben mit den jeweiligen Gewichtungen entspricht. Aus Darstellungsgründen sind die unterschiedlichen Farben als unterschiedliche Schraffuren dargestellt.

Die Gewichtung jeder Farbe wird durch die Summe oder den Durchschnittswert der gespeicherten und/oder momentan empfangenen Puls-Signale bestimmt, die in eine Gruppe eingeteilt werden. Die drei Puls-Signale der einzelnen Messungen werden im unteren Bereich auf einer relativen Skala dargestellt. In jedem Block 55, 57, 59, 61 von drei Puls-Signalen ist rechts die cun-Position, mittig die guan-Position und links die chi-Position dargestellt. Die Blöcke 55, 57 links werden/wurden an der linken Hand gemessen und die Blöcke 59, 61 rechts an der rechten Hand. Die oberen Blöcke 55, 59 sind bei niedrigem Messdruck (oberflächlich/Yang) aufgenommen und die unteren 57, 61 bei hohem Messdruck (tief/Yin). Entsprechend der Zuordnung der Pulsdiagnose zur Fünf-Elemente-Lehre werden die maximalen Signalamplituden die an der chi-Position gemessenen Puls-Signale der rechten Hand und die an der cun-Position gemessenen Puls-Signale der linken Hand in eine Gruppe mit der Bezeichnung "Feuer" eingeteilt und rot dargestellt. Die maximalen Signalamplituden der guan-Position der linken Hand sind grün und werden in die Gruppe "Holz" eingeteilt. Die maximalen Signalamplituden der guan-Position der rechten Hand sind braun und werden in die Gruppe "Erde" eingeteilt. Die maximalen Signalamplituden der chi-Position der linken Hand sind blau und werden in die Gruppe "Wasser" eingeteilt. Die maximalen Signalamplituden der cun-Position der rechten Hand sind silber/weiß und werden in die Gruppe "Metall" eingeteilt.

Das Maximum der relativen Skala, auf der die Balkenanzeige für die maximalen Signalamplituden dargestellt ist, kann durch das Puls-Signal mit der höchsten maximalen Signalamplitude bestimmt werden und das Minimum der Skala kann durch das Puls-Signal mit der niedrigsten maximalen Signalamplitude bestimmt werden. Das bedeutet, dass sich auch die Darstellung der gespeicherten Puls-Signale ändern kann, wenn sich die Skala verändert. Mittels eines Intensitätsreglers 63 kann eine Signalverstärkung eingestellt werden, um die Messung qualitativ zu kalibrieren. Es versteht sich, dass die Signalverstärkung nicht zwischen einzelnen Messungen der zwölf Puls-Signale verändert werden sollte.

Der Touch-Screen des Anzeigemittels 21 weist außerdem mehrere Befehls-Felder 65, 67, 69, 71 und 73 auf. Das Befehlsfeld 65 mit einen Dreieckssymbol kann gedrückt werden, um einen Abspielmodus zu starten. Das Befehlsfeld 67 mit einen Kreissymbol kann gedrückt werden, um einen Aufnahmemodus zu starten bzw. zu stoppen. Das Befehlsfeld 69 mit einen Lautsprechersymbol kann gedrückt werden, um ein Audiosignal über einen Audio-Ausgang oder einen Lautsprecher auszugeben. Das Befehlsfeld 71 mit der Aufschrift "5 Elemente" kann gedrückt werden, um eine andere Gruppeneinteilung entsprechend einer anderen Diagnosemethode zu wählen und einen entsprechenden Visualisierungsmodus zu starten. Die Aufschrift entspricht dem jeweils aktuell gewählten Visualisierungsmodus bzw. der aktuell gewählten Diagnosemethode. Durch Drücken des Befehlsfelds 73 mit der Aufschrift "Speichern" kann eine momentane Visualisierung gespeichert werden. Die jeweils zuletzt gemessene Pulsrate 75 wird ebenfalls auf dem Touch-Screen des Anzeigemittels 21 in der Einheit "Schläge pro Minute" (englisch: "beats per minute", bpm) angezeigt.

Das erfindungsgemäße Verfahren bzw. die erfindungsgemäße Vorrichtung hat den Vorteil, dass eine Mehrzahl von Puls-Signalen übersichtlich dargestellt werden kann und die behandelnde Person leicht alle Puls-Signale auf einem Blick miteinander vergleichen kann. Im Falle einer Pulsdiagnose nach TCM können beispielsweise die maximalen Signalamplituden von 12 Puls-Signalen nebeneinander angezeigt werden, die an den drei Körpermesspunkten *chi*, *guan* und *cun* am Handgelenk jeder Hand mit den jeweiligen Messdrücken oberflächig und tief gemessen wurden bzw. werden.

## Patentansprüche

1. Verfahren zur Visualisierung einer Mehrzahl von Puls-Signalen mit den Schritten:
- Empfangen mindestens eines ersten Puls-Signals der Mehrzahl von Puls-Signalen,
- Visualisieren einer quantifizierbaren Eigenschaft des mindestens einen ersten Puls-Signals auf einem Anzeigemittel (21),
**dadurch gekennzeichnet, dass** das Verfahren ferner die Schritte aufweist:
- Speichern der quantifizierbaren Eigenschaft des mindestens einen ersten Puls-Signals,
- Empfangen mindestens eines zweiten Puls-Signals der Mehrzahl von Puls-Signalen, und
- Zuordnen des mindestens einen ersten Puls-Signals und des mindestens einen zweiten Puls-Signals jeweils zu einem bestimmten Körpermesspunkt und/oder Messdruck,
und **dadurch gekennzeichnet, dass**
die quantifizierbare Eigenschaft des mindestens einen ersten Puls-Signals auf dem Anzeigemittel (21) visualisiert wird, indem die gespeicherte quantifizierbare Eigenschaft des mindestens einen ersten Puls-Signals und die quantifizierbare Eigenschaft des mindestens einen zweiten Puls-Signals jeweils an einer für die Zuordnung zu einem Körpermesspunkt und/oder Messdruck charakteristischen Stelle auf dem Anzeigemittel (21) visualisiert werden.

2. Verfahren nach Anspruch 1, wobei die quantifizierbare Eigenschaft die maximale Signalamplitude ist.

3. Verfahren nach Anspruch 2, wobei die Signalamplituden der Mehrzahl von Puls-Signalen jeweils in Bezug auf eine Skala visualisiert werden, wobei das Maximum der Skala durch das Puls-Signal mit der höchsten maximalen Signalamplitude bestimmt wird und das Minimum der Skala durch das Puls-Signal mit der niedrigsten maximalen Signalamplitude bestimmt wird.

4. Verfahren nach einem der vorhergehenden Ansprüche, wobei das Visualisieren der quantifizierbaren Eigenschaft des mindestens einen zweiten Puls-Signals vor dem Ende des Empfangens des mindestens einen zweiten Puls-Signals begonnen wird.

5. Verfahren nach einem der vorhergehenden Ansprüche, wobei die Mehrzahl von Puls-Signalen in eine oder mehrere Gruppen eingeteilt werden und die Summe und/oder der Durchschnittswert der quantifizierbaren Eigenschaft der Puls-Signale jeder Gruppe jeweils an einer für die Gruppe charakteristischen Stelle auf dem Anzeigemittel (21) visualisiert wird.

6. Verfahren nach Anspruch 5, wobei jeder Gruppe eine für die Gruppe charakteristische Farbe und/oder Bezeichnung zugeordnet wird und die Summe und/oder der Durchschnittswert der quantifizierbaren Eigenschaft der Puls-Signale jeder Gruppe jeweils in der für die Gruppe charakteristischen Farbe und/oder mit der für die Gruppe charakteristischen Bezeichnung auf dem Anzeigemittel (21) visualisiert wird.

7. Verfahren nach Anspruch 5 oder 6, wobei der Summe und/oder dem Durchschnittswert der quantifizierbaren Eigenschaft der Puls-Signale einer Gruppe ein Audiosignal zugeordnet wird und das Audiosignal über einen Audio-Ausgang oder einen Lautsprecher ausgegeben wird.

8. Vorrichtung zur Visualisierung einer Mehrzahl von Puls-Signalen mit
- einem Puls-Signalverarbeitungsmittel (19), das dazu eingerichtet ist, mindestens ein erstes Puls-Signal der Mehrzahl von Puls-Signalen zu empfangen, und
- einem Anzeigemittel (21), das dazu eingerichtet ist, eine quantifizierbare Eigenschaft des mindestens einen ersten Puls-Signals auf einem Anzeigemittel zu visualisieren,
**dadurch gekennzeichnet, dass**
das Puls-Signalverarbeitungsmittel (19) ferner dazu eingerichtet ist, die quantifizierbare Eigenschaft des mindestens einen ersten Puls-Signals zu speichern, mindestens ein zweites Puls-Signal der Mehrzahl von Puls-Signalen zu empfangen, und das mindestens eine erste Puls-Signal und das mindestens eine zweite Puls-Signal jeweils einem bestimmten Körpermesspunkt und/oder Messdruck zuzuordnen, und
das Anzeigemittel (21) ferner dazu eingerichtet ist, die quantifizierbare Eigenschaft des mindestens einen ersten Puls-Signals auf dem Anzeigemittel (21) zu visualisieren, indem die gespeicherte quantifizierbare Eigenschaft des mindestens einen ersten Puls-Signals und die quantifizierbare Eigenschaft des mindestens einen zweiten Puls-Signals jeweils an einer für die Zuordnung zu einem Körpermesspunkt und/oder Messdruck charakteristischen Stelle auf dem Anzeigemittel (21) visualisiert werden.

9. Vorrichtung nach Anspruch 8, wobei das Anzeigemittel (21) ein Farbdisplay aufweist.

10. Vorrichtung nach Anspruch 8 oder 9, wobei das Puls-Signalverarbeitungsmittel (19) und das Anzeigemittel (21) dazu eingerichtet sind, das Visualisieren der quantifizierbaren Eigenschaft des mindestens einen zweiten Puls-Signals vor dem Ende des Empfangens des mindestens einen zweiten Puls-Signals zu beginnen.

11. Vorrichtung nach einem der Ansprüche 8 bis 10, ferner mit einem mit dem Puls-Signalverarbeitungsmittel (19) kommunikativ verbundenen Puls-Signalerfassungsmittel (23), das dazu eingerichtet ist, gleichzeitig den Puls an drei nebeneinander liegenden Körpermesspunkten zu messen.

12. Vorrichtung nach Anspruch 11, wobei das Puls-Signalerfassungsmittel (23) drei in einer Reihe angeordnete Drucksensoren (29a, 29b, 29c) aufweist, wobei das Puls-Signalerfassungsmittel (23) dazu eingerichtet ist, den Puls bei mindestens zwei verschiedenen reproduzierbaren Messdrücken zu messen.

13. Vorrichtung nach einem der Ansprüche 8 bis 12, wobei das Puls-Signalverarbeitungsmittel (23) einen Audio-Eingang (41) aufweist, der dazu eingerichtet ist, die Puls-Signale zu empfangen.

14. Vorrichtung nach einem der Ansprüche 8 bis 13, wobei das Puls-Signalverarbeitungsmittel (23) einen Audio-Ausgang oder Lautsprecher aufweist, der dazu eingerichtet ist, ein für die quantifizierbare Eigenschaft der empfangenen und/oder gespeicherten Puls-Signale zugeordnetes Audiosignal auszugeben.

15. Maschinen-lesbares Medium mit gespeicherten Anweisungen zur Ausführung der Verfahrensschritte gemäß einem der Ansprüche 1 bis 7.
